# EUROPÄISCHE PATENTANMELDUNG

(11) **EP 3 778 855 A1**
(43) Veröffentlichungstag der Anmeldung: **17.02.2021**
(21) Anmeldenummer: 19191473.8
(22) Anmeldetag: 13.08.2019
(51) Int. Cl.: C12M 1/26, C12P 3/00, C02F 11/04

(54) **FILTERBASIERTE REGENERATION VON ANAEROBSCHLÄMMEN**

(71) Anmelder: Axiom Angewandte Prozeßtechnik Ges. m.b.H., 2483 Ebreichsdorf (AT)
(72) Erfinder: WENGER-OEHN, Hermann, 4880 St. Georgen im Attergau (AT)
(74) Vertreter: Sonn & Partner Patentanwälte

(57) **Zusammenfassung**

Die vorliegende Erfindung betrifft ein Verfahren zur Behandlung eines methanisierenden Schlammes, wobei der methanisierende Schlamm über einen mit einer Deckschicht belegten mechanischen Filter mit einer Belebungszone enthaltend nitrifizierende Mikroorganismen in Kontakt gebracht wird, wobei die nitrifizierenden Mikroorganismen durch den mit der Deckschicht belegten mechanischen Filter in der Belebungszone gehalten werden. Des Weiteren betrifft die Erfindung eine Vorrichtung zur Durchführung des erfindungsgemäßen Verfahrens.

## Beschreibung

Die vorliegende Erfindung betrifft ein Verfahren zur Behandlung eines methanisierenden Schlammes.

Die Aufbereitung von methanisierenden Schlämmen, wie beispielsweise von Faulschlämmen aus Kläranlagen und Gärresten aus Biogasanlagen, ist von zentraler Bedeutung für deren Aktivität und Verwertung, beispielsweise als landwirtschaftlicher Dünger, oder Entsorgung.

Vor der Verwertung oder Entsorgung werden methanisierende Schlämme üblicherweise entwässert, unter anderem um die Volumina zu reduzieren. Aktive methanisierende anaerobe Schlämme können bei der Vergärung ein starkes negatives Potential im Bereich von - 400 mVolt entwickeln. Gründe sind hier unter anderem die Gasgleichgewichte, insbesondere von Kohlendioxid, und die negativ geladenen Dissoziationspartner in der wässrigen Phase, wie das Hydrogencarbonat. Diese negativ geladenen Stoffe können durch Wechselwirkungen mit dem unspezifischen Gemenge an Abbauprodukten in den Schlämmen, insbesondere Ammoniumstickstoff, in der wässrigen Phase gehalten werden. Ein starkes negatives Potential kann den Anteil an gebundenem Wasser erhöhen und so die Entwässerbarkeit verringern. Dies macht den Einsatz großer Mengen von Konditionierungsmittel, üblicherweise von kationischen Polymeren, zum Flocken des Schlammes erforderlich. Der Polymerbedarf wird dabei vor allem von der negativen Ladungsmenge des Schlammes bestimmt.

Für den Einsatz als landwirtschaftlicher Dünger kann ein hoher Stickstoffgehalt ebenfalls unerwünscht sein. In vielen Regionen gibt es durch intensive Landwirtschaft und Tierhaltung bereits eine hohe Stickstoffversorgung der Böden und ein zusätzlicher Nährstoffeintrag kann eine erhöhte Eutrophierung der Gewässer und Belastung des Grundwassers mit sich bringen.

Ein weiteres Problem von Ammoniumstickstoff in methanisierenden Schlämmen ist, dass bei hohen Konzentrationen die Mikrobiologie gehemmt werden kann. Somit kann es zu einem reduzierten Abbau der eingesetzten Abfälle kommen und in weiterer Folge beispielsweise zu einem instabilen Biogasprozess.

Neben dem Ammoniumstickstoff ist häufig auch ein hoher Gehalt an löslichen Kohlenstoffverbindungen unerwünscht. Insbesondere im Fall von Kläranlagen müssen Kohlenstoffverbindungen, häufig in Form von im Abwasser enthaltenen Schmutzstoffen, gemessen als CSB, abgebaut werden.

Einige Verfahren zur Entfernung von Ammoniumstickstoff und anderen Stoffen aus Faulschlämmen, Gärresten oder anderen Substraten sind aus dem Stand der Technik bekannt.

Die WO 2011/137466 A1 offenbart ein Verfahren, in welchem Ammoniak bzw. Ammonium mittels einer Entspannungsverdampfung reduziert, von den Biogasanlagen-Fermentationsflüssigkeiten oder - Gärresten abgetrennt und als Ammoniak abgeschieden wird.

Die US 2003/0057162 A1 offenbart Verfahren zur Reduzierung von Verunreinigungen in Form von elektrisch geladenen Verbindungen aus wässrigen Medien. Das wässrige Medium wird dabei entlang einer elektrisch geladenen Membran geleitet, die für die zu entfernende Verunreinigung durchlässig ist, jedoch im Wesentlichen undurchlässig ist für ungeladene oder entgegengesetzt geladene Verbindungen. Auf der anderen Seite der Membran befindet sich ein wässriges Reaktionsmedium enthaltend Mikroorganismen zum Abbau der Verunreinigung.

Die WO 2013/0105854 A1 offenbart ein Verfahren zur Stickstoffgewinnung aus einer Ammonium enthaltenden Flüssigkeit. Ammoniumionen werden durch eine Ionenaustauschmembran von einer Anodenkammer in eine Kathodenkammer transportiert, wo H⁺-Ionen verbraucht werden und durch einen Anstieg des pH-Werts Ammoniak beispielsweise durch Spülen mit einem Inertgas extrahiert werden kann.

Die EP 0 458 241 A2 beschreibt Verfahren und Vorrichtungen zur Abtrennung von Ammonium aus wässrigen Flüssigkeiten. Die ammoniumhaltige Flüssigkeit ist durch eine Kationenaustauschmembran von einer "akzeptorseitigen" Lösung getrennt, deren pH-Wert gegenüber der "Donorseite" erhöht ist. Durch den höheren pH-Wert wird auf der Akzeptorseite vermehrt NH₃ gebildet, welches beispielsweise durch Unterdruck und/oder Hindurchleiten von Inertgas fortlaufend entfernt werden kann, sodass quer zur Membran ein NH₄⁺-Konzentrationsgefälle aufrechterhalten wird.

Die WO 2014/026015 A1 betrifft biologische Behandlungssysteme mit selektiv durchlässigen Barrieren zur biologischen Beseitigung von Verunreinigungen aus wässrigen Medien, insbesondere für die Aquakultur. Offenbart wird unter anderem eine Ausführungsform, in welcher eine Gärkammer durch eine selektive Ionenaustauschmembran, die für Nitrat (NO₃⁻) nicht aber für Ammonium (NH₄⁺) durchlässig ist, die von einem Nitrifizierungsreaktor getrennt ist. Ammoniumhaltiges Medium wird aus der Gärkammer in den Nitrifizierungsreaktor gepumpt, worauf NH₃ nitrifiziert wird und als Nitrat in die Gärkammer diffundiert. Das Medium mit einer nun reduzierten Konzentration an Ammoniumstickstoff wird in der Folge aus dem Nitrifizierungsreaktor abgeleitet. Das Nitrat kann dabei in der Gärkammer denitrifiziert werden.

Die WO 2015/066021 A1 offenbart Systeme zur Behandlung von Abwasser, insbesondere aus Aquakulturen, enthaltend zwei Kammern, die durch eine semipermeable Barriere, vorzugsweise eine Ionenaustauschmembran, voneinander getrennt sind. In beschriebenen Ausführungsformen wird Abwasser mit hohem NH₃-Gehalt in beide Kammern geleitet, wobei eine der beiden Kammern ein Nitrifizierungsreaktor ist. Die selektive Membran zwischen den Kammern erlaubt den Durchtritt von NO₃⁻ vom Nitrifizierungsreaktor in die zweite Kammer, wo das NO₃⁻ denitrifiziert werden kann. Die Membran verhindert aber den Transport von NH₃ bzw. NH₄⁺. Medium mit reduzierter Stickstoffkonzentration wird in der Folge aus dem Nitrifizierungsreaktor abgeleitet.

Die im Stand der Technik beschriebenen Verfahren zur Reduzierung von Ammoniumstickstoff oder anderer Verunreinigungen aus Schlämmen oder anderen Substraten weisen Nachteile in Bezug auf die Effektivität und/oder auf die Komplexität der Verfahren auf. Es besteht daher weiterhin ein Bedarf an Verfahren zur Behandlung von methanisierenden Schlämmen, welche es ermöglichen die Konzentration von Ammoniumstickstoff und/oder Kohlenstoffverbindungen zu reduzieren und die technische Behandelbarkeit, insbesondere die Entwässerbarkeit, der Schlämme sowie die Aktivität von Anaerobreaktoren zu verbessern. Die Aufgabe der vorliegenden Erfindung besteht darin, ein solches Verfahren zur Verfügung zu stellen.

Die vorliegende Erfindung betrifft daher ein Verfahren zur Behandlung eines methanisierenden Schlammes, wobei der methanisierende Schlamm über einen mit einer Deckschicht belegten mechanischen Filter mit einer Belebungszone enthaltend nitrifizierende Mikroorganismen in Kontakt gebracht wird, wobei die nitrifizierenden Mikroorganismen durch den mit der Deckschicht belegten mechanischen Filter in der Belebungszone gehalten werden.

Die vorliegende Erfindung betrifft außerdem eine Vorrichtung zur Durchführung des erfindungsgemäßen Verfahrens, aufweisend eine Schlammzone zur Aufnahme des methanisierenden Schlammes und eine Belebungszone enthaltend nitrifizierende Mikroorganismen, wobei die Schlammzone und die Belebungszone durch einen mechanischen Filter voneinander getrennt sind.

Gemäß dem erfindungsgemäßen Verfahren ist der mechanische Filter mit einer Deckschicht belegt. Diese Deckschicht kann sich durch das Einbringen des methanisierenden Schlammes in die Schlammzone bilden. Im Zusammenhang mit der vorliegenden Erfindung soll unter dem Begriff "Deckschicht" jegliche Belegung des mechanischen Filters verstanden werden, welche Belegung Bestandteile des methanisierenden Schlammes umfasst. Vorzugsweise ist die Deckschicht ein Biofilm. Ein solcher Biofilm kann durch die Belegung des Filters mit Mikroorganismen aus dem methanisierenden Schlamm entstehen. Es kann sich bei der Deckschicht aber auch um eine anorganische Belegung, beispielsweise durch Salze, handeln. Vorzugsweise vermindert die Deckschicht die Durchlässigkeit des mechanischen Filters für Mikroorganismen, insbesondere für nitrifizierende Bakterien. Vorzugsweise ist die Deckschicht allerdings für geladene und ungeladene kleine Moleküle, insbesondere für Ammonium (NH₄⁺), Ammoniak (NH₃) und Nitrat (NO₃⁻ ), durchlässig.

Der mit der Deckschicht belegte mechanische Filter (in der Folge auch als Filterbarriere bezeichnet) ermöglicht die Trennung des zu behandelnden Schlammes in der Schlammzone von den nitrifizierenden Mikroorganismen (beispielsweise in der Form von Belebtschlamm) in der Belebungszone. Gleichzeitig ermöglicht diese Filterbarriere den Austausch von unterschiedlich geladenen oder ungeladenen kleinen Molekülen wie Ammonium (NH₄⁺), Ammoniak (NH₃) oder Nitrat (NO₃⁻) zwischen den Zonen. NH₄⁺ und/oder NH₃ kann somit von der Schlammzone in die Belebungszone diffundieren, wo durch Nitrifikation durch die dort vorliegenden Mikroorganismen NO₃⁻ gebildet wird. NO₃⁻ kann durch die Filterbarriere zurück in die Schlammzone diffundieren, wo eine Denitrifikation durch im zu methanisierenden Schlamm vorliegende Mikroorganismen stattfinden und molekularer Stickstoff (N₂) gebildet werden kann. Durch die Denitrifikation wird neben dem Stickstoffgehalt auch der Gehalt an Kohlenstoffverbindungen im methanisierenden Schlamm erheblich reduziert, was unter anderem für Kläranlagen einen erheblichen Vorteil darstellt.

Somit ermöglicht die vorliegende Erfindung die Kombination einer Nitrifikation, welche die Anwesenheit von Sauerstoff voraussetzt, mit einer Denitrifikation, welche vorzugsweise unter Abwesenheit von Sauerstoff abläuft, zur Entfernung von Ammoniumstickstoff sowie Kohlenstoff aus dem methanisierenden Schlamm. Dies wird insbesondere dadurch ermöglicht, dass die Filterbarriere einen mechanischen Filter umfasst, der sowohl für positiv (insbesondere NH₄⁺) als auch für negativ (insbesondere NO₃⁻) geladene Ionen durchlässig ist. Durch die Belegung der Oberfläche des mechanischen Filters infolge der Deckschichtbildung werden jedoch die anaerobe Biomasse in der Schlammzone und die nitrifizierende Biomasse in der Belebungszone gehalten.

Im Zusammenhang mit der vorliegenden Erfindung ist unter dem Begriff "methanisierender Schlamm" jedes Produkt einer anaeroben Vergärung, insbesondere einer mikrobiellen Methanisierung, von zumindest teilweise biogenem Material zu verstehen. Dabei kann bei Anwendung des erfindungsgemäßen Verfahrens die Vergärung noch im Gange sein oder bereits abgeschlossen sein. Der methanisierende Schlamm ist vorzugsweise Faulschlamm, insbesondere aus einer Kläranlage, oder Gärrest, insbesondere aus einer Biogasanlage.

Vorzugsweise liegen die nitrifizierenden Mikroorganismen in der Belebungszone in Form von Belebtschlamm vor. Belebtschlamm bezeichnet eine Ansammlung an Mikroorganismen, die unter aeroben Bedingungen die Nitrifikation durchführen können. Die Mikroorganismen sind vorzugsweise ausgewählt aus Bakterien, Pilzen und Protozoen.

In einer bevorzugten Ausführungsform weist der mechanische Filter eine Porengröße zwischen 5 und 200 µm, bevorzugt zwischen 7,5 und 150 µm, noch mehr bevorzugt zwischen 10 und 100 µm, am meisten bevorzugt zwischen 15 und 75 µm auf. In Kombination mit der Deckschichtbildung wird dadurch eine Filterbarriere ermöglicht, die für kleine Moleküle (insbesondere für NH₃, NH₄⁺ und NO₃⁻) durchlässig ist, jedoch die nitrifizierenden Mikroorganismen in der Belebungszone halten kann. Vorzugsweise weist der mechanische Filter eine NH₄⁺/NO₃⁻-Permselektivität zwischen 0,2 und 5, bevorzugt zwischen 0,4 und 2,5, noch mehr bevorzugt zwischen 0,5 und 2, insbesondere zwischen 0,7 und 1,5, am meisten bevorzugt zwischen 0,8 und 1,25 auf. Vorzugsweise ist der mechanische Filter nicht elektrisch geladen. Bei dem mechanischen Filter gemäß der Erfindung handelt es sich nicht um eine Ionenaustauschmembran.

Im Rahmen der Erfindung ist darunter, dass die Mikroorganismen durch den mit der Deckschicht belegten mechanischen Filter in der Belebungszone gehalten werden, zu verstehen, dass es keinen freien Austausch der Mikroorganismen zwischen der Belebungszone und der Schlammzone gibt. Es ist allerdings nicht so zu verstehen, dass der Austausch zu 100 % verhindert wird und keine einzelnen Mikroorganismen von der Belebungszone in die Schlammzone übertreten können. Bei der Durchführung des Verfahrens ist es bevorzugt, wenn nach einer Stunde noch mindestens 80 Gew.-%, vorzugsweise mindestens 90 Gew.-%, noch mehr bevorzugt mindestens 95 Gew.-%, am meisten bevorzugt mindestens 99 Gew.-% der in die Belebungszone eingebrachten Mikroorganismen bzw. des in die Belebungszone eingebrachten Belebtschlammes noch in der Belebungszone vorliegen. Im Rahmen der Erfindung ist es außerdem bevorzugt, wenn nach einer Stunde noch mindestens 80 Gew.-%, vorzugsweise mindestens 90 Gew.-%, noch mehr bevorzugt mindestens 95 Gew.-%, am meisten bevorzugt mindestens 99 Gew.-% des in die Schlammzone eingebrachten methanisierenden Schlammes noch in der Schlammzone vorliegen.

In einer bevorzugten Ausführungsform des erfindungsgemäßen Verfahrens wird der Belebungszone Sauerstoff zugeführt. Da die Nitrifikation in der Belebungszone unter der Verwendung von Sauerstoff abläuft, führt die Zuführung von Sauerstoff zu einem effizienten Ablauf der Nitrifikation. Der Sauerstoff kann dabei über ein Belüftungselement zugeführt werden. Im Zusammenhang mit der erfindungsgemäßen Vorrichtung ist es daher bevorzugt, dass die Belebungszone ein Belüftungselement aufweist.

Die in der Belebungszone ablaufende Nitrifikation kann beispielsweise durch folgendes Reaktionsschema dargestellt werden: NH₃ + 2 O₂ → NO₃⁻ + H⁺ + H₂O. Eine in der Schlammzone ablaufende Denitrifikation kann beispielsweise durch folgendes Schema dargestellt werden: 2 NO₃⁻ + 10 e⁻ + 12 H⁺ → N₂ + 6 H₂O. Als Elektronenquelle (e⁻) können dabei Kohlenstoffverbindungen aus dem methanisierenden Schlamm oder zusätzlich zugesetzte Substrate wie z.B. Methanol dienen.

Wenn bei einem Verfahren gemäß der Erfindung in der Belebungszone eine Nitrifikation und in der Schlammzone eine Denitrifikation stattfindet, kommt es in der Belebungszone zu einer Senkung des pH-Werts (Produktion von H⁺) und in der Schlammzone zu einem Anstieg des pH-Werts (Verbrauch von H⁺). Im Rahmen der Erfindung ist es daher bevorzugt, dass der methanisierende Schlamm einen pH-Wert von über 7,0, vorzugsweise über 7,5, noch mehr bevorzugt über 8,0, am meisten bevorzugt über 8,5 aufweist. Außerdem ist es bevorzugt, wenn das Medium in der Belebungszone einen pH-Wert von unter 7,0, vorzugsweise unter 6,5, noch mehr bevorzugt unter 6,0, am meisten bevorzugt unter 5,5 aufweist. Besonders bevorzugt ist es, wenn das Medium in der Belebungszone einen niedrigeren pH-Wert als der methanisierende Schlamm aufweist.

In einer bevorzugten Ausführungsform der Erfindung wird der methanisierende Schlamm über einen weiteren mit einer Deckschicht belegten mechanischen Filter mit einer Pufferzone in Kontakt gebracht. Definitionen und bevorzugte Ausführungsformen für die erste Deckschicht auf dem Filter zwischen Schlammzone und Belebungszone gelten in gleichem Maße auch für diese Deckschicht. Durch diese Ausführungsform wird ermöglicht, dass NH₃ aus der Schlammzone in die Pufferzone diffundiert, wobei der methanisierende Schlamm in der Schlammzone gehalten wird. Dadurch kann der Gehalt an Ammoniumstickstoff im methanisierenden Schlamm zusätzlich reduziert werden. Außerdem kann durch den Austausch von Medium zwischen der Pufferzone und der Schlammzone sichergestellt werden, dass sich der pH-Wert in der Schlammzone nicht zu stark in das Alkalische verschiebt, was zu einer Hemmung der dort stattfindenden Reaktionen, insbesondere der Denitrifikation, führen könnte. In Bezug auf die erfindungsgemäße Vorrichtung ist es daher bevorzugt, dass die Vorrichtung eine Pufferzone aufweist, welche durch einen weiteren mechanischen Filter von der Schlammzone getrennt ist.

Die Pufferzone enthält vorzugsweise ein wässriges Medium. Vorzugsweise weist das Medium in der Pufferzone einen niedrigeren pH-Wert als der methanisierende Schlamm auf. Alle bevorzugten Ausführungsformen des mechanischen Filters, welcher erfindungsgemäß zwischen Schlammzone und Belebungszone vorgesehen ist, sind auch für den mechanischen Filter zwischen der Pufferzone und der Schlammzone bevorzugt.

In einer bevorzugten Ausführungsform wird Ammoniak (NH₃) aus der Pufferzone abgezogen. Dadurch kann das aus der Schlammzone in die Pufferzone aufgenommene NH₃ rückgewonnen und weiter verwertet werden. Vorzugsweise wird das NH₃ durch Anlegen eines Unterdruckes in der Pufferzone gewonnen.

In einer weiteren bevorzugten Ausführungsform wird Medium von der Belebungszone abgezogen, entsalzen, und über ein Verbindungselement in die Pufferzone geleitet. Zweckmäßigerweise erfolgt die Entsalzung durch eine Umkehrosmose. Vorzugsweise handelt es sich um eine Vollentsalzung. Da das aus der Belebungszone abgezogene Medium vorzugsweise einen niedrigeren pH-Wert als die Schlammzone aufweist, kann in dieser Ausführungsform eine Senkung des pH-Werts in der Pufferzone (welche mit der Schlammzone in Wechselwirkung steht) erreicht werden.

Es hat sich im Rahmen der Erfindung als besonders zweckmäßig erwiesen, wenn das Medium aus der Belebungszone über eine getauchte Membran abgezogen wird. Dadurch kann sichergestellt werden, dass die nitrifizierenden Mikroorganismen in der Belebungszone verbleiben. Zum Abziehen des Mediums aus der Belebungszone kann ein gewöhnliches Verfahren zur Mikrofiltration eingesetzt werden. Im Zusammenhang mit der Vorrichtung gemäß der Erfindung ist es bevorzugt, dass die Vorrichtung ein Verbindungselement zwischen der Belebungszone und der Pufferzone aufweist, wobei das Verbindungselement durch eine Membran von der Belebungszone getrennt ist.

Als Membran kann dabei eine Membran, wie sie üblicherweise für die Mikrofiltration eingesetzt wird, verwendet werden. Vorzugsweise weist die Membran eine Porengröße zwischen 0,02 und 50 µm, bevorzugt zwischen 0,1 und 10 µm, insbesondere zwischen 0,2 und 5 µm auf. Vorzugsweise handelt es sich um eine ungeladene Membran.

In einer weiteren bevorzugten Ausführungsform der vorliegenden Erfindung wird Medium von der Pufferzone über ein (gegebenenfalls weiteres) Verbindungselement in die Belebungszone geleitet. Im Zusammenhang mit der erfindungsgemäßen Vorrichtung ist es daher bevorzugt, dass die Vorrichtung ein Verbindungselement zwischen der Pufferzone und der Belebungszone aufweist. Dadurch, dass die Pufferzone mit der Schlammzone in Wechselwirkung steht, welche vorzugsweise einen höheren pH-Wert als die Belebungszone aufweist, kann auch das Medium in der Pufferzone einen höheren pH-Wert als das Medium in der Belebungszone aufweisen. Diese Ausführungsform hat somit den Vorteil, dass der pH-Wert in der Belebungszone angehoben werden und eine Hemmung der Nitrifikation durch einen zu niedrigen pH-Wert verhindert werden kann. Somit wird eine Stabilisierung des pH-Werts erreicht.

Im Rahmen der Erfindung hat es sich als besonders zweckmäßig erwiesen, wenn sowohl Medium aus der Belebungszone in die Pufferzone geleitet wird, als auch Medium aus der Pufferzone zurück in die Belebungszone geleitet wird. Somit kann Medium kontinuierlich zwischen der Belebungszone und der Pufferzone zirkuliert werden, wobei der durch die Nitrifikation in der Belebungszone niedrigere pH-Wert in der Pufferzone durch die Wechselwirkung mit der Schlammzone ansteigen kann. Vorzugsweise liegen das Verbindungselement zur Leitung von Medium von der Belebungszone in die Pufferzone und das Verbindungselement zur Leitung von Medium aus der Pufferzone in die Belebungszone an entgegengesetzten Enden der Pufferzone. Dadurch kann eine effiziente Wechselwirkung mit der Schlammzone erreicht werden.

In einer weiteren bevorzugten Ausführungsform wird der Belebungszone Wasser zugeführt. Dadurch kann verdunstetes und/oder abgezogenes Wasser ergänzt werden. Außerdem ist es bevorzugt, wenn der Belebungszone Base oder Säure zur pH-Regulation zugeführt wird.

Im Folgenden wird die Erfindung anhand von bevorzugten, nicht einschränkenden Ausführungsformen unter Bezugnahme auf die Zeichnungen näher erläutert.
Figur 1 zeigt eine schematische Darstellung des Austausches von NH₃, NH₄⁺ und NO₃⁻ zwischen der Schlammzone und der Belebungszone sowie Reaktionsschemata von in den jeweiligen Zonen ablaufenden chemischen Reaktionen.
Figur 2 zeigt ein Fließschema einer bevorzugten Ausführungsform des erfindungsgemäßen Verfahrens.

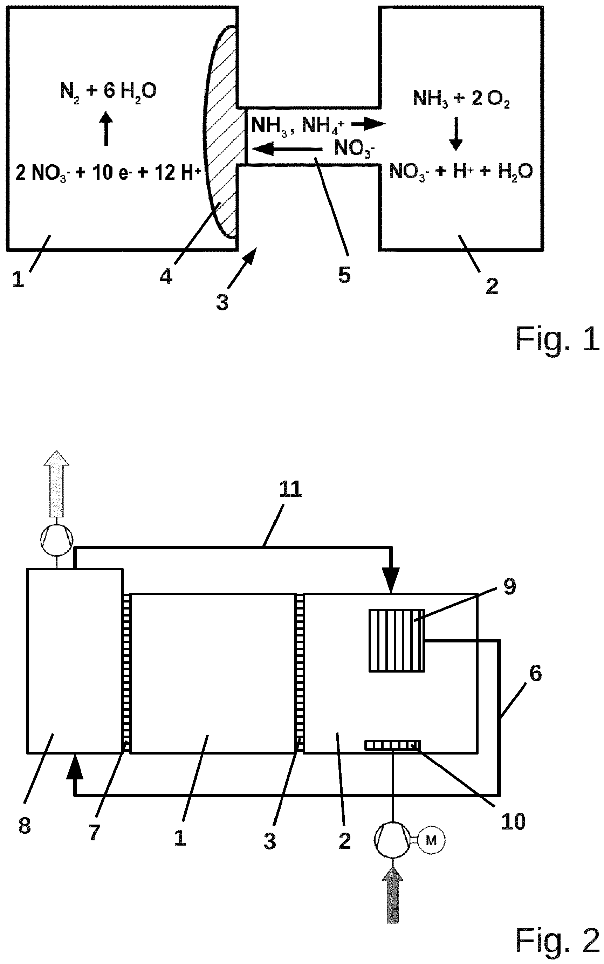

Figur 1 zeigt schematisch den Austausch von NH₃, NH₄⁺ und NO₃⁻ zwischen der Schlammzone 1 und der Belebungszone 2 über einen mechanischen Filter 3, welcher mit einer Deckschicht 4 belegt ist. Ammoniumstickstoff in Form von NH₃ und NH₄⁺ diffundiert durch die Filterpore 5 in die Belebungszone 2. In der Belebungszone 2 wird Ammoniumstickstoff durch die dort vorliegenden nitrifizierenden Mikroorganismen zu NO₃⁻ umgewandelt. NO₃⁻ kann wiederum durch die Filterpore 5 in die Schlammzone 1 diffundieren, wo es in einer Denitrifikation zu N₂ umgewandelt wird.

Figur 2 zeigt eine bevorzugte Ausführungsform des erfindungsgemäßen Verfahrens. Der methanisierende Schlamm wird über einen mit einer Deckschicht belegten mechanischen Filter 3 mit einer Belebungszone 2 enthaltend nitrifizierende Mikroorganismen in Kontakt gebracht. Die nitrifizierenden Mikroorganismen werden dabei durch den mit der Deckschicht (nicht dargestellt) belegten mechanischen Filter 3 in der Belebungszone 2 gehalten. Die Belebungszone 2 enthält ein Belüftungselement 6 zur Zufuhr von Sauerstoff. In der gezeigten Ausführungsform wird der methanisierende Schlamm über einen weiteren mit einer Deckschicht (nicht dargestellt) belegten mechanischen Filter 7 mit einer Pufferzone 8 in Kontakt gebracht. Medium wird durch Mikrofiltration über eine getauchte Membran 9 aus der Belebungszone 2 abgezogen, entsalzen und über ein Verbindungselement 10 in die Pufferzone 8 geleitet. Aus der Pufferzone 8 wird wiederum Medium über ein weiteres Verbindungselement 11 in die Belebungszone 2 geleitet.

In der gezeigten Ausführungsform können NH₃ und NH₄⁺ aus der Schlammzone 1 in die Belebungszone 2 diffundieren. Der in der Belebungszone 2 vorliegende Belebtschlamm bewirkt eine Nitrifikation und somit eine Umwandlung von NH₃ und NH₄⁺ zu NO₃⁻. Durch Einsetzen der Nitrifikation verschiebt sich der pH-Wert in der Belebungszone 2 in den sauren Bereich. Das gebildete Nitrat diffundiert wiederum in die Schlammzone 1, wo unter Verbrauch von Kohlenstoff als Elektronenquelle eine Denitrifikation erfolgt, welche den pH-Wert in der Schlammzone 1 in das Alkalische verschiebt. In der gezeigten Ausführungsform wird ein Teil des sauren Wassers aus der Belebungszone 2 abgezogen und nach Entsalzung über das Verbindungselement 6 in die Pufferzone 8 geleitet. Das in die Pufferzone 8 geleitete saure und entsalzte Wasser kann nun mit der Schlammzone 1 wechselwirken und unter anderem NH₃ und NH₄⁺ aufnehmen. Durch die Wechselwirkung mit dem alkalischen Medium in der Schlammzone 1 steigt der pH-Wert des in die Pufferzone 8 geleiteten Wassers, welches zur pH-Stabilisierung durch das Verbindungselement 11 in die Belebungszone 2 geleitet wird. In der gezeigten Ausführungsform gibt es somit eine kontinuierliche Zirkulation von Medium zwischen der Belebungszone 2 und der Pufferzone 8, wobei der niedrige pH-Wert des aus der Belebungszone 2 abgezogenen Mediums in der Pufferzone 8 durch Wechselwirkung mit dem alkalischen Medium in der Schlammzone 1 angehoben wird.

Durch die Kombination von Nitrifikation und Denitrifikation sinkt die Konzentration von Ammoniumstickstoff im behandelten methanisierenden Schlamm. Zusätzlich führt die Denitrifikation dazu, dass auch der Kohlenstoffgehalt reduziert wird. Außerdem sorgt ein leichter Unterdruck in der Pufferzone 8 dafür, dass aus der Schlammzone 1 in die Pufferzone 2 übergegangener Ammoniumstickstoff beispielsweise durch Regulation des pH-Wertes als NH₃ abgesaugt und so wiedergewonnen werden kann. Durch die Reduzierung von Ammoniumstickstoff im methanisierenden Schlamm sinkt weiters die Ladungsdichte, wodurch die technische Behandelbarkeit verbessert wird und der Polymerbedarf zur Entwässerung sinkt.

## Patentansprüche

1. Verfahren zur Behandlung eines methanisierenden Schlammes, **dadurch gekennzeichnet, dass** der methanisierende Schlamm über einen mit einer Deckschicht (4) belegten mechanischen Filter (3) mit einer Belebungszone (2) enthaltend nitrifizierende Mikroorganismen in Kontakt gebracht wird, wobei die nitrifizierenden Mikroorganismen durch den mit der Deckschicht (4) belegten mechanischen Filter (3) in der Belebungszone (2) gehalten werden.

2. Verfahren gemäß Anspruch 1, **dadurch gekennzeichnet, dass** der Belebungszone (2) Sauerstoff zugeführt wird.

3. Verfahren gemäß Anspruch 1 oder 2, **dadurch gekennzeichnet, dass** der methanisierende Schlamm über einen weiteren mit einer Deckschicht belegten mechanischen Filter (7) mit einer Pufferzone (8) in Kontakt gebracht wird.

4. Verfahren gemäß einem der vorhergehenden Ansprüche, **dadurch gekennzeichnet, dass** Medium von der Belebungszone (2) abgezogen wird, entsalzen wird, und über ein Verbindungselement (6) in die Pufferzone (8) geleitet wird.

5. Verfahren gemäß einem der vorhergehenden Ansprüche, **dadurch gekennzeichnet, dass** Medium von der Pufferzone (8) über ein Verbindungselement (11) in die Belebungszone (2) geleitet wird.

6. Verfahren gemäß einem der vorhergehenden Ansprüche, **dadurch gekennzeichnet, dass** Ammoniak aus der Pufferzone (8) abgezogen wird.

7. Verfahren gemäß einem der vorhergehenden Ansprüche, **dadurch gekennzeichnet, dass** der methanisierende Schlamm einen pH-Wert von über 7,0 aufweist.

8. Verfahren gemäß einem der vorhergehenden Ansprüche, **dadurch gekennzeichnet, dass** das Medium in der Belebungszone (2) einen pH-Wert von unter 7,0 aufweist.

9. Verfahren gemäß einem der vorhergehenden Ansprüche, **dadurch gekennzeichnet, dass** das Medium in der Belebungszone (2) einen niedrigeren pH-Wert als der methanisierende Schlamm aufweist.

10. Vorrichtung zur Durchführung des Verfahrens gemäß einem der vorhergehenden Ansprüche, aufweisend eine Schlammzone (1) zur Aufnahme des methanisierenden Schlammes und eine Belebungszone (2) enthaltend nitrifizierende Mikroorganismen, wobei die Schlammzone (1) und die Belebungszone (2) durch einen mechanischen Filter (3) voneinander getrennt sind.

11. Vorrichtung gemäß Anspruch 10, **dadurch gekennzeichnet, dass** der mechanische Filter (3) eine Porengröße zwischen 5 und 200 µm aufweist.

12. Vorrichtung gemäß Anspruch 10 oder 11, **dadurch gekennzeichnet, dass** die Belebungszone (2) ein Belüftungselement (10) aufweist.

13. Vorrichtung gemäß einem der Ansprüche 10 bis 12, **dadurch gekennzeichnet, dass** die Vorrichtung eine Pufferzone (8) aufweist, welche durch einen weiteren mechanischen Filter (7) von der Schlammzone (1) getrennt ist.

14. Vorrichtung gemäß Anspruch 13, **dadurch gekennzeichnet, dass** die Vorrichtung ein Verbindungselement (6) zwischen der Belebungszone (2) und der Pufferzone (8) aufweist, wobei das Verbindungselement (6) durch eine Membran (9) von der Belebungszone (2) getrennt ist.

15. Vorrichtung gemäß Anspruch 13 oder 14, **dadurch gekennzeichnet, dass** die Vorrichtung ein Verbindungselement (11) zwischen der Pufferzone (8) und der Belebungszone (2) aufweist.
